# EUROPEAN PATENT APPLICATION

(11) **EP 3 572 099 A1**
(43) Date of publication of application: **27.11.2019**
(21) Application number: 18205167.2
(22) Date of filing: 08.11.2018
(51) Int. Cl.: A61L 12/08, C11D 3/00, G02C 13/00, G02C 7/04, G02B 1/04, A61L 12/14

(54) **OPHTHALMIC COMPOSITION**

(30) Priority: 25.05.2018 TW 107117948
(71) Applicant: Pegavision Corporation, Taoyuan City 333 (TW)
(72) Inventor: TING, Wei-Jia, 333 Taoyuan City (TW); KUAN, Ting-Chun, 333 Taoyuan City (TW); LU, Shu-Chen, 333 Taoyuan City (TW)
(74) Representative: Viering, Jentschura & Partner mbB Patent- und Rechtsanwälte

(57) **Abstract**

An ophthalmic composition includes effective amounts of a comfort enhancing agent, a surfactant, and a buffering agent. The comfort enhancing agent includes a first functional monomer that is 2-methacryloyloxyethyl phosphorylcholine (MPC). The comfort enhancing agent has a HLB value between 8 and 40. In addition, the comfort enhancing agent can further include a second functional monomer that is preferably n-butyl methacrylate (BMA).

## Description

### CROSS-REFERENCE TO RELATED PATENT APPLICATION

This application claims the benefit of priority to Taiwan Patent Application No. 107117948, filed on May 25, 2018. The entire content of the above identified application is incorporated herein by reference.

Some references, which may include patents, patent applications and various publications, may be cited and discussed in the description of this disclosure. The citation and/or discussion of such references is provided merely to clarify the description of the present disclosure and is not an admission that any such reference is "prior art" to the disclosure described herein. All references cited and discussed in this specification are incorporated herein by reference in their entireties and to the same extent as if each reference was individually incorporated by reference.

### FIELD OF THE DISCLOSURE

The present disclosure relates to an ophthalmic product and an ophthalmic composition thereof.

### BACKGROUND OF THE DISCLOSURE

3C products such as smartphones and computers are frequently used in today's information society, resulting in an increase in the myopia population and a decrease in the age group with myopia. In consideration of user convenience and aesthetics, it is generally a good choice for people with myopia to wear contact lenses.

Since most people with myopia need to wear contact lenses for a long period of time, it is very important to improve the wearing comfort of contact lenses. The wearing comfort of contact lenses is associated with oxygen permeability, moisture retention, and lubricity of the contact lenses. Although the material of the contact lenses have been constantly improved to enhance oxygen permeability, moisture retention, and lubricity, the wearers thereof, in different use environments or with different eye conditions, the eyes of user may still suffer from corneal injury or lesions caused by corneal hypoxia and dehydration with an increase of wear-time, especially in an air-conditioned room for a long period of time.

Since contact lens solutions provided with glycerol would only reduce or relieve the discomfort, there is a need for a novel ophthalmic composition used on contact lenses to increase the wearing comfort of the contact lens and maintain the cornea in a healthy state.

### SUMMARY OF THE DISCLOSURE

In response to the above-referenced technical inadequacies, the present disclosure provides an ophthalmic product and an ophthalmic composition thereof.

In one aspect, the present disclosure provides an ophthalmic composition, including effective amounts of a comfort enhancing agent, a surfactant, and a buffering agent. The comfort enhancing agent includes a first functional monomer that is 2-methacryloyloxyethyl phosphorylcholine (MPC). The comfort enhancing agent has a HLB value between 8 and 40.

In one aspect, the present disclosure provides an ophthalmic product, including a packaging container and a contact lens packaged in the packaging container. The packaging container has an aforesaid ophthalmic composition in aqueous solution form stored therein. The contact lens is immersed in direct contact with the ophthalmic composition.

In certain embodiments, the comfort enhancing agent includes a second functional monomer that is an acrylic acid ester or a methacrylic acid ester having a C2-C20 alkyl group.

In certain embodiments, the second functional monomer includes one or a combination of two or more of n-butyl methacrylate (BMA), 2-ethylhexyl methacrylate (EHMA), isodecyl methacrylate (IDMA), lauryl methacrylate (LMA), 2-hydroxyethyl methacrylate (HEMA), and 2,3-dihydroxypropyl methacrylate (DHPM).

In certain embodiments, the second functional monomer is n-butyl methacrylate (BMA).

In certain embodiments, the effective amount of the comfort enhancing agent is from 0.01 wt % to 3 wt % based on a total of 100 wt % of the ophthalmic composition.

In certain embodiments, the comfort enhancing agent has a number average molecular weight between 100 Daltons and 500,000 Daltons.

In certain embodiments, the surfactant is a polysorbate 80 surfactant, an alkyl sulfosuccinate surfactant, or a combination thereof.

In certain embodiments, the effective amount of the surfactant is from 0.01 wt % to 2 wt % based on a total of 100 wt % of the ophthalmic composition.

In certain embodiments, the buffering agent is a borate buffering agent or a phosphate buffering agent.

In certain embodiments, the borate buffering agent includes one or a combination of two or more of boric acid, sodium chloride, and sodium tetraborate. The phosphate buffering agent includes one or a combination of two or more of sodium chloride, monobasic sodium phosphate, dibasic sodium phosphate, monobasic potassium phosphate, dibasic potassium phosphate.

In certain embodiments, the effective amount of the buffering agent is from 0.01 wt % to 5 wt % based on a total of 100 wt % of the ophthalmic composition.

In certain embodiments, the ophthalmic composition further includes at least one hydrophilic molecule that includes one or a combination of two or more of polyvinyl alcohol, cellulose and its derivatives, and hyaluronic acid.

In certain embodiments, the effective amount of the at least one hydrophilic molecule is from 0.01 wt % to 10 wt % based on a total of 100 wt % of the ophthalmic composition.

In certain embodiments, the ophthalmic composition further includes at least one functional additive that is an antimicrobial agent, a vitamin, or a combination thereof.

In certain embodiments, the effective amount of the at least one functional additive from 0.01 wt % to 5 wt % based on a total of 100 wt % of the ophthalmic composition.

In certain embodiments, the ophthalmic composition has a pH value between 6 and 8 and an osmolality between 250 osmol/Kg and 450 osmol/Kg.

One of the advantages of the instant disclosure is that the ophthalmic product and the ophthalmic composition use the technical solution of "the comfort enhancing agent includes a first functional monomer that is 2-methacryloyloxyethyl phosphorylcholine (MPC)" to increase the wearing comfort of the contact lens and maintain the cornea in a healthy state.

These and other aspects of the present disclosure will become apparent from the following description of the embodiment taken in conjunction with the following drawings and their captions, although variations and modifications therein may be affected without departing from the spirit and scope of the novel concepts of the disclosure.

### BRIEF DESCRIPTION OF THE DRAWINGS

The present disclosure will become more fully understood from the detailed description and the accompanying drawings, in which:
FIG. 1 is a perspective view of an exemplary example of an ophthalmic product according to the present disclosure.
FIG. 2 is a schematic side view of the exemplary example of the ophthalmic product according to the present disclosure.
FIG. 3 is a flow chart of a method for using the ophthalmic composition according to the present disclosure.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an", and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

Unless defined otherwise, technical and scientific terms used herein have the same meaning as is commonly understood by one of skill in the art to which this invention belongs. As used herein, the singular forms "a", "an" and "the" are intended to include the plural forms as well. Unless specific definitions are provided, the laboratory procedures and techniques described herein are those well-known and commonly used in the art. These procedures and techniques are performed according to conventional methods as described in various general references.

Unless otherwise indicated, all percentages used herein are by weight. When a series of lower limit ranges and a series of upper limit ranges are provided, all combinations of the provided ranges are contemplated as if each combination were specifically listed.

In order to increase wearing comfort of the contact lens, the present disclosure provides an ophthalmic composition that can be applied to an ophthalmic preparation or related products of contact lenses. Examples of the ophthalmic preparation include an eye drop, an eye wash, and an ophthalmic medicament. The related product can be a product used to treat contact lenses for various purposes, and examples thereof include a storage solution, a washing solution, and a disinfection solution. The ophthalmic composition of the present disclosure includes effective amounts of a comfort enhancing agent, a surfactant, and a buffering agent. Water can be used as a dispersant in the ophthalmic composition, but is not limited thereto. Water is present in an amount from 75 wt % to 99 wt %, preferably from 85 wt % to 99 wt %, based on a total of 100 wt % of the ophthalmic composition.

In the present embodiment, the comfort enhancing agent at least includes a first functional monomer, i.e., the comfort enhancing agent can be a homopolymer of the first functional monomer. The first functional monomer has good hydrophilicity and is 2-methacryloyloxyethyl phosphorylcholine (MPC). In addition, the comfort enhancing agent can further include a second functional monomer, i.e., the comfort enhancing agent can be a copolymer of the first and second functional monomers and include repeating units each composed of the first and second functional monomers that are linked with each other. The second functional monomer has good water solubility, dispersibility, and water absorption and transport properties, and is an acrylic acid ester or a methacrylic acid ester having a C2-C20 alkyl group. The effective amount of the comfort enhancing agent is from 0.01 wt % to 3 wt %, preferably from 0.01 wt % to 1.5 wt %, based on a total of 100 wt % of the ophthalmic composition. The comfort enhancing agent has a number average molecular weight between 100 Daltons and 500,000 Daltons. The comfort enhancing agent has a HLB (hydrophilic-lipophilic balance) value between 8 and 40.

Specific examples of the methacrylic acid ester having a C2-C20 alkyl group include: n-butyl methacrylate (BMA), 2-ethylhexyl methacrylate (EHMA), isodecyl methacrylate (IDMA), lauryl methacrylate (LMA), 2-hydroxyethyl methacrylate (HEMA), and 2,3-dihydroxypropyl methacrylate (DHPM). In other words, the comfort enhancing agent that is suitable for being used in the present disclosure can be poly(MPC-co-BMA), poly(MPC-co-EMHA), poly(MPC-co-IDMA), poly(MPC-co-LMA), poly(MPC-co-HEMA), or poly(MPC-co-DHPM), preferably poly(MPC-co-BMA).

It was discovered that when a contact lens is immersed in a solution of the ophthalmic composition, ingredients in the solution enter the contact lens or adhere onto the surface of the contact lens. Accordingly, a thin film of the comfort enhancing agent having good oxygen permeability, surface hydrophilicity, moistability, and lubricity can be formed on the surface of the contact lens. Therefore, the wearing comfort of the contact lens, especially during long-term contact with eyes, can be increased.

The following will further describe the reason that the first functional monomer uses MPC. In the tear film of human eyes, the innermost layer is in direct contact with the cornea and the main ingredient thereof is water; the outermost layer is exposed to the air and includes phospholipids each containing a portion of the structure of a phosphorylcholine group. Therefore, the quick evaporation of water in the innermost layer can be avoided and the dryness of the cornea can be prevented. If in the ophthalmic composition, the first functional monomer of the comfort enhancing agent has the phosphorylcholine group, it would achieve good moisture retaining effect that is similar to the outermost layer of the human tear film and increase the stability of the human tear film.

In other embodiments, the comfort enhancing agent can further includes a third functional monomer, i.e., the comfort enhancing agent can be a copolymer of the first, second and third functional monomers and include repeating units each composed of the first, second and third functional monomers that are linked together. The third functional monomer can be a hydrophilic monomer such as methacrylic acid (MA) and a vinyl monomer containing carboxyl groups (e.g., CH₂=CH(CH₃)C(O)OC₂H₄OC(O)C₂H₄COOH (MS)), but is not limited thereto. In other words, the comfort enhancing agent can be poly(MPC-co-BMA -co-MA) or poly(MPC-co-BMA-co-MS).

The surfactant is a polysorbate 80 surfactant (TWEEN 80™), an alkyl sulfosuccinate surfactant (GEROPON™), or a combination thereof. The surfactant is adapted to clean the contact lens, i.e., remove lipids and other deposits (e.g., proteins) from the surface of the contact lens. In addition, the comfort enhancing agent can enhance the expression of the surfactant. For example, the comfort enhancing agent can keep the eye moist for a long period of time and increase the tear exchange rate. The effective amount of the surfactant is from 0.01 wt % to 2 wt %, preferably from 0.01 wt % to 1 wt %, based on a total of 100 wt % of the ophthalmic composition.

The buffering agent can be a borate buffering agent or a phosphate buffering agent. The buffering agent is adapted to adjust the pH and osmolality of the ophthalmic composition, so that the ophthalmic composition can achieve the expected effect. The effective amount of the buffering agent is from 0.01 wt % to 5 wt % based on a total of 100 wt % of the ophthalmic composition. Preferably, the ophthalmic composition has a pH value between 6 and 8 and an osmolality between 250 osmol/Kg and 450 osmol/Kg. The borate buffering agent can include one or a combination of two or more of boric acid, sodium chloride, and sodium tetraborate, but is not limited thereto. The phosphate buffering agent can include one or a combination of two or more of sodium chloride, monobasic sodium phosphate, dibasic sodium phosphate, monobasic potassium phosphate, dibasic potassium phosphate, but is not limited thereto.

In addition to the above main ingredients, the ophthalmic composition can further include at least one hydrophilic molecule that is adapted to moisten the contact lens. The at least one hydrophilic molecule can include one or a combination of two or more of polyvinyl alcohol (PVA), cellulose and its derivatives, polyethylene glycol (PEG), and hyaluronic acid (HA), but is not limited thereto. Examples of cellulose derivatives include hydroxypropyl methylcellulose (HPMC), carboxymethyl cellulose (CMC), methyl cellulose (MC), hydroxyethyl cellulose (HEC), and cationic cellulose derivatives. The effective amount of the at least one hydrophilic molecule is from 0.01 wt % to 5 wt %, preferably from 0.01 wt % to 3 wt %, based on a total of 100 wt % of the ophthalmic composition.

It was discovered that the specific hydrophilic molecule(s) helps the contact lens retain beneficial ingredients thereon, so as to prevent the beneficial ingredients from being brought away from the contact lens by tears. More specifically, in the ophthalmic composition of the present disclosure, each of the ingredients may have a different capability of adhesion, introduction or penetration relative to the contact lens due to the material properties (e.g., hydrophilicity and crosslink density) of the contact lens and the polar differences between the ingredients and the contact lens. It should be noted that the specific hydrophilic molecule(s) can interact with the beneficial ingredients to increase their adhesion capability, so as to allow the contact lens to retain as much of the beneficial ingredients as possible. Therefore, when the user wears the contact lenses, the beneficial ingredients would be stably released from the contact lenses and then transferred into the cornea. For example, the comfort enhancing agent can achieve the effects of moistening the cornea and helping retaining moisture in the cornea.

The ophthalmic composition of the present disclosure can further include at least one functional additive depending on requirements so as to increase its applicability. The at least one functional additive can include a vitamin, an antimicrobial agent, or a combination thereof, but is not limited thereto. The effective amount of the at least one functional additive from 0.01 wt % to 5 wt % based on a total of 100 wt % of the ophthalmic composition. Examples of the vitamin include vitamin B6 (pyridoxine hydrochloride), vitamin B12 (cyanocobalamin), and vitamin E (synthetic dl-alpha-tocopherol). Examples of the antimicrobial agent include polyhexamethylene biguanide (PHMB) and its water soluble salts and polyaminopropyl biguanide(PAPB) and its water soluble salts.
Representative embodiments of the ophthalmic composition of the present disclosure are listed in Table 1 and Table 2.

**Table 1**

| ingredients | Example 1 | Example 2 | Example 3 | Example 4 | Example 5 | Example 6 |
|---|---|---|---|---|---|---|
| boric acid | 0.50% | 0.50% | 0.50% | 0.80% | 0.80% | 0.80% |
| sodium chloride | 0.30% | 0.30% | 0.30% | 0.60% | 0.60% | 0.60% |
| sodium tetraborate | 0.03% | 0.03% | 0.03% | 0.05% | 0.05% | 0.05% |
| TWEEN 80™ | 0.50% | 0.50% | 0.50% | 0.50% | 0.01% | 0.01 % |
| polyvinyl alcohol | | | | 0.50% | 5.00% | 5.00% |
| hyaluronic acid | | | | | 3.00% | |
| sodium hyaluronate (HA-LF-P) | | | | | | 1.50% |
| sodium hyaluronate (HA-LF5-A) | | | | | | 1.50% |
| sodium hyaluronate (HA-LQ) | | | | | | |
| vitamin B6 | | | | | | |
| vitamin E | | | | | | |
| vitamin B12 | | | | | | |
| poly(MPC) | 0.20% | 0.20% | | 1.00% | 0.05% | 0.05% |
| poly(MPC-co- BMA) | | 0.20% | 0.20% | 1.00% | 0.05% | 0.01% |

**Table 2**

| ingredients | Example 7 | Example 8 | Example 9 | Example 10 | Example 11 |
|---|---|---|---|---|---|
| boric acid | 0.80% | 0.50% | 0.50% | 0.70% | 0.70% |
| sodium chloride | 0.60% | 0.30% | 0.30% | 0.50% | 0.50% |
| sodium tetraborate | 0.05% | 0.03% | 0.03% | 0.10% | 0.10% |
| TWEEN 80™ | 0.01% | 0.50% | 0.50% | 0.10% | 0.10 % |
| polyvinyl alcohol | 5.00% | | | 2.00% | 2.00% |
| hyaluronic acid | 1.00% | | | 1.00% | 1.00% |
| sodium hyaluronate (HA-LF-P) | 1.00% | | | | |
| sodium hyaluronate (HA-LF5-A) | | | | | |
| sodium hyaluronate (HA-LQ) | 1.00% | | | 1.00% | 1.00% |
| vitamin B6 | | 0.30% | 0.30% | 0.30% | 0.05% |
| vitamin E | | | 0.30% | 0.30% | 0.01% |
| vitamin B12 | | 0.30% | 0.30% | 0.30% | 0.05% |
| poly(MPC) | 0.01% | 0.50% | 0.50% | 1.00% | 1.00% |
| poly(MPC-co-BMA) | 0.01% | 0.50% | 0.50% | 1.00% | 1.00% |

In Tables 1 and 2, the ophthalmic compositions of Examples 1-3 include only basic ingredients (i.e., a comfort enhancing agent, a surfactant, and a buffering agent). The ophthalmic composition of Example 4, compared with that of Examples 1-3, further includes a hydrophilic molecule. The ophthalmic compositions of Examples 5-7, compared with that of Examples 1-3, further include at least two different hydrophilic molecules. The ophthalmic compositions of Examples 8 and 9, compared with that of Examples 1-3, further include vitamins. The ophthalmic compositions of Examples 10 and 11, compared with that of Examples 1-3, further include one or more hydrophilic molecules and vitamins.

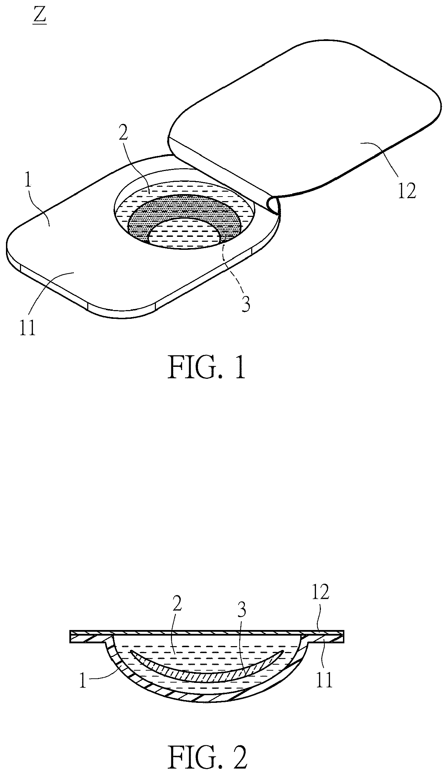

Referring to FIGS. 1 and 2, the ophthalmic composition of the present disclosure is preferably used as a contact lens storage solution/packaging solution. Accordingly, the present disclosure provides an ophthalmic product Z (i.e., a contact lens product) which includes a packaging container 1, an aqueous ophthalmic composition 2, and a contact lens 3. The aqueous ophthalmic composition 2 is sealed with the contact lens 3 in the packaging container 1, wherein the contact lens 3 is immersed in the aqueous ophthalmic composition 2. In practice, a sterilization process such as a high temperature or high pressure sterilization process is performed on the ophthalmic product Z.

More specifically, the packaging container 1 includes a blister pack 11 that can provide a certain protection to the contact lens 3 and a peelable covering sheet 12 made of metal or plastic. The contact lens 3 can be a daily disposable contact lens, being worn by a user when he/she is awake and removed from his/her eyes before sleep to be disposed of. The contact lens 3 can also be an extended wear contact lens, which can be worn by a user over 7 days, 14 days or more and stored in the aqueous ophthalmic composition 2 every night for supplementation of beneficial the ingredient(s) such as the comfort enhancing agent.

The contact lens 3 can be a silicone hydrogel contact lens that helps improve oxygen permeability so as to prevent red eyes, bloodshot eyes, and redness in the eye caused by corneal hypoxia. The silicone hydrogel contact lens can be made of materials classified by the U.S. FDA in Group V as: Balafilcon A, Comfilcon A, Efrofilcon A, Enfilcon A, Galyfilcon A, Lotrafilcon A, Lotrafilcon B, Narafilcon A, Narafilcon B, Senofilcon A, Delefilcon A, and Somofilcon A. Furthermore, other ingredients such as blue light and ultraviolet absorbing ingredients can be added to the silicone hydrogel material depending on the requirements.

The contact lens 3 can also be a hydrogel contact lens that helps improve wettability and lubricity so as to increase wearing comfort. The hydrogel contact lens can be made of nonionic polymers having a low water content of less than 50 wt %, classified by the U.S. FDA. in Group I as: Helfilcon A&B, Hioxifilcon B, Mafilcon, Polymacon, Tefilcon, and Tetrafilcon A. The hydrogel contact lens can also be made of nonionic polymers having a high water content of greater than 50 wt %, classified by the U.S. FDA in Group II as: Acofilcon A, Alfafilcon A, Hilafilcon B, Hioxifilcon A, Hioxifilcon B, Hioxifilcon D, Nelfilcon A, Nesofilcon A, Omafilcon A, and Samfilcon A. The hydrogel contact lens can be made of ionic polymers having a low water content of less than 50 wt %, classified by the U.S. FDA in Group III as Deltafilcon A. The hydrogel contact lens can also be made of ionic polymers having a high water content of greater than 50 wt %, classified by the U.S. FDA in GroupIV as: Etafilcon A, Focofilcon A, Methafilcon A, Methafilcon B, Ocufilcon A, Ocufilcon B, Ocufilcon C, Ocufilcon D, Ocufilcon E, Phemfilcon A, and Vifilcon A.

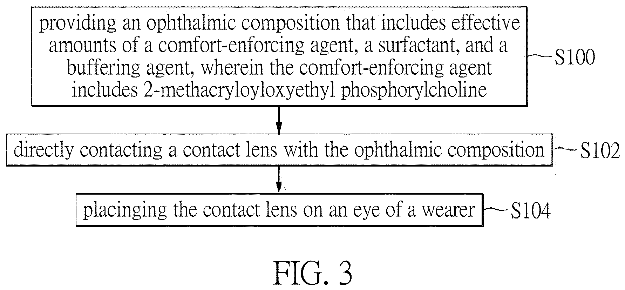

Reference is now made to FIG. 3. The ophthalmic composition of the present disclosure can be used to wash contact lenses. Accordingly, the present disclosure further provides a method for using the ophthalmic composition, including: step S100, providing an ophthalmic composition that includes effective amounts of a comfort enhancing agent, a surfactant, and a buffering agent, wherein the comfort enhancing agent includes 2-methacryloyloxyethyl phosphorylcholine; step S102, directly contacting a contact lens with the ophthalmic composition; and step S104, placing the contact lens on an eye of a wearer. The term "direct contact" refers to immersing the contact lens in the ophthalmic composition or dispense drops the ophthalmic composition onto the contact lens.

### Experiment 1: Preparation of Ophthalmic Products

Contact lens storage solutions were prepared according to the compositions of Examples 1, 4, 5, 7, 9 and 10 shown in Tables 1 and 2. Hydrogel contact lenses (water content: 58%) manufactured by PEGAVISION CORPORATION were respectively immersed in the contact lens storage solutions. After the sealing and sterilization processes, the ophthalmic products were obtained, in which the surface characteristics of the hydrogel contact lenses were shown in Table 3.

**Table 3**

| | debris adhesion | breakage | Surface lubricity |
|---|---|---|---|
| contact lens immersed in solution of Example 1 | ⊚ | ⊚ | × |
| contact lens immersed in solution of Example 4 | ⊚ | ⊚ | Δ |
| contact lens immersed in solution of Example 5 | ⊚ | ⊚ | Δ |
| contact lens immersed in solution of Example 7 | ⊚ | ⊚ | ⊚ |
| contact lens immersed in solution of Example 9 | ⊚ | ⊚ | Δ |
| contact lens immersed in solution of Example 10 | ⊚ | ⊚ | ⊚ |

| | | | |
|---|---|---|---|
| "⊚" indicates no debris/no breakage/excellent surface lubricity "Δ" indicates a relatively small amount of debris/breakage having a relatively small area/good surface lubricity "×" indicates a relatively large amount of debris/breakage having a relatively large area/bad surface lubricity | | | |

### Experiment 2: Water Film Break-up-time (WBUT) Test

Contact lens storage solutions were prepared according to the compositions of Examples 1, 4, 5, 7, 9 and 10 shown in Tables 1 and 2. Hydrogel contact lenses (water content: 58%) manufactured by PEGAVISION CORPORATION were respectively immersed in the contact lens storage solutions. These contact lenses were taken out from the solutions and their water-film break up times were recorded and shown in Table 4.

**Table 4**

| | WBUT (seconds) |
|---|---|
| contact lens being immersed in solution of Example 1 | 45 |
| contact lens being immersed in solution of Example 4 | 47 |
| contact lens being immersed in solution of Example 5 | 49 |
| contact lens being immersed in solution of Example 7 | 52 |
| contact lens being immersed in solution of Example 9 | 46 |
| contact lens being immersed in solution of Example 10 | 55 |

### Experiment 3: Water Evaporation Rate Test

Contact lens storage solutions were prepared according to the compositions of Examples 1, 4, 5, 7, 9 and 10 shown in Tables 1 and 2. Hydrogel contact lenses (water content: 58%) manufactured by PEGAVISION CORPORATION were respectively immersed in the contact lens storage solutions. These contact lenses were taken out from the solutions and their water contents at different times were recorded and shown in Tables 5 and 6.

**Table 5**

| | contact lens being immersed in solution of Example 1 | contact lens being immersed in solution of Example 4 | contact lens being immersed in solution of Example 5 |
|---|---|---|---|
| water content after 1 hour | 37% | 39% | 41% |
| water content after 2 hours | 5% | 6% | 10% |
| water content after 3 hours | 3% | 3% | 5% |
| water content after 4 hours | 1% | 2% | 3% |

**Table 6**

| | contact lens being immersed in solution of Example 7 | contact lens being immersed in solution of Example 9 | contact lens being immersed in solution of Example 10 |
|---|---|---|---|
| water content after 1 hour | 43% | 38% | 44% |
| water content after 2 hours | 11% | 6% | 13% |
| water content after 3 hours | 5% | 3% | 6% |
| water content after 4 hours | 5% | 1% | 5% |

### Experiment 5: Wearing Sensation Test

Contact lens storage solutions were prepared according to the compositions of Examples 1, 4, 5, 7, 9 and 10 shown in Tables 1 and 2. Hydrogel contact lenses (water content: 58%) manufactured by PEGAVISION CORPORATION were respectively immersed in the contact lens storage solutions. These contact lenses were taken out from the solutions and then put on the user's eyes. Scores for each of the items of comfort, wettability, and lubricity, as shown in Tables 7 and 8, are average scores calculated from five users.

**Table 7**

| evaluation items | wear-in time | self-feeling score | | |
|---|---|---|---|---|
| | | contact lens being immersed in solution of Example 1 | contact lens being immersed in solution of Example 4 | contact lens being immersed in solution of Example 5 |
| comfort sensation | immediately right after | ⊚ | ⊚ | ⊚ |
| | after 1 hour | × | Δ | Δ |
| | after 8 hours | × | × | × |
| moisture sensation | immediately right after | Δ | Δ | ⊚ |
| | after 1 hour | Δ | Δ | Δ |
| | after 8 hours | × | Δ | Δ |
| lubrication sensation | immediately right after | Δ | ⊚ | ⊚ |
| | after 1 hour | Δ | Δ | Δ |
| | after 8 hours | × | Δ | Δ |

| | | | | |
|---|---|---|---|---|
| "⊚" indicates an excellent sensation "Δ" indicates a good sensation "×" indicates a bad sensation | | | | |

**Table 8**

| evaluation items | wear-in time | self-feeling score | | |
|---|---|---|---|---|
| | | contact lens being immersed in solution of Example 7 | contact lens being immersed in solution of Example 9 | contact lens being immersed in solution of Example 10 |
| comfort sensation | immediately right after | ⊚ | ⊚ | ⊚ |
| | after 1 hour | ⊚ | ⊚ | ⊚ |
| | after 8 hours | Δ | Δ | Δ |
| moisture sensation | immediately right after | ⊚ | ⊚ | ⊚ |
| | after 1 hour | Δ | Δ | ⊚ |
| | after 8 hours | Δ | Δ | Δ |
| lubrication sensation | immediately right after | ⊚ | ⊚ | ⊚ |
| | after 1 hour | Δ | Δ | ⊚ |
| | after 8 hours | Δ | Δ | Δ |

| | | | | |
|---|---|---|---|---|
| "⊚" indicates an excellent sensation "Δ" indicates a good sensation "×" indicates a bad sensation | | | | |

One of the advantages of the instant disclosure is that the ophthalmic product and the ophthalmic composition use the technical solution of "the comfort enhancing agent includes a first functional monomer that is 2-methacryloyloxyethyl phosphorylcholine (MPC)" to increase wearing comfort of the contact lens and maintain the cornea in a healthy state.

Furthermore, a thin film of the comfort enhancing agent can be formed on the surface of the contact lens and has good oxygen permeability, surface hydrophilicity, wettability, and lubricity. Therefore, the wearing comfort of the contact lens, especially after long-term contact with the eye, can be increased. The comfort enhancing agent can further include a second functional monomer that is preferably n-butyl methacrylate (BMA) so as to be highly expressed in the ophthalmic composition.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its spirit and scope.

## Claims

1. An ophthalmic composition, comprising effective amounts of a comfort enhancing agent, a surfactant, and a buffering agent, wherein the comfort enhancing agent includes a first functional monomer that is 2-methacryloyloxyethyl phosphorylcholine (MPC), and the comfort enhancing agent has a HLB value between 8 and 40.

2. The ophthalmic composition according to claim 1, wherein the comfort enhancing agent includes a second functional monomer that is an acrylic acid ester or a methacrylic acid ester having a C2-C20 alkyl group.

3. The ophthalmic composition according to claim 2, wherein the second functional monomer includes one or a combination of two or more of n-butyl methacrylate (BMA), 2-ethylhexyl methacrylate (EHMA), isodecyl methacrylate (IDMA), lauryl methacrylate (LMA), 2-hydroxyethyl methacrylate (HEMA), and 2,3-dihydroxypropyl methacrylate (DHPM).

4. The ophthalmic composition according to claim 3, wherein the second functional monomer is n-butyl methacrylate (BMA).

5. The ophthalmic composition according to claim 1, wherein the effective amount of the comfort enhancing agent is from 0.01 wt % to 3 wt % based on a total of 100 wt % of the ophthalmic composition.

6. The ophthalmic composition according to claim 1, wherein the comfort enhancing agent has a number average molecular weight between 100 Daltons and 500,000 Daltons.

7. The ophthalmic composition according to claim 1, wherein the surfactant is a polysorbate 80 surfactant, an alkyl sulfosuccinate surfactant, or a combination thereof.

8. The ophthalmic composition according to claim 1, wherein the effective amount of the surfactant is from 0.01 wt % to 2 wt % based on a total of 100 wt % of the ophthalmic composition.

9. The ophthalmic composition according to claim 1, wherein the buffering agent is a borate buffering agent or a phosphate buffering agent.

10. The ophthalmic composition according to claim 9, wherein the borate buffering agent includes one or a combination of two or more of boric acid, sodium chloride, and sodium tetraborate, and wherein the phosphate buffering agent includes one or a combination of two or more of sodium chloride, monobasic sodium phosphate, dibasic sodium phosphate, monobasic potassium phosphate, dibasic potassium phosphate.

11. The ophthalmic composition according to claim 10, wherein the effective amount of the buffering agent is from 0.01 wt % to 5 wt % based on a total of 100 wt % of the ophthalmic composition.

12. The ophthalmic composition according to claim 1, further comprising at least one hydrophilic molecule that includes one or a combination of two or more of polyvinyl alcohol, cellulose and its derivatives, and hyaluronic acid.

13. The ophthalmic composition according to claim 12, wherein the effective amount of the at least one hydrophilic molecule is from 0.01 wt % to 10 wt % based on a total of 100 wt % of the ophthalmic composition.

14. The ophthalmic composition according to claim 1, further comprising at least one functional additive that is an antimicrobial agent, a vitamin, or a combination thereof.

15. The ophthalmic composition according to claim 14, wherein the effective amount of the at least one functional additive from 0.01 wt % to 5 wt % based on a total of 100 wt % of the ophthalmic composition.

16. The ophthalmic composition according to claim 1, having a pH value between 6 and 8 and an osmolality between 250 osmol/Kg and 450 osmol/Kg.
